(19) Europäisches Patentamt / European Patent Office / Office européen des brevets

(11) **EP 3 234 823 B1**

(12) **EUROPEAN PATENT SPECIFICATION**

(45) Date of publication and mention
of the grant of the patent:
**25.03.2020  Bulletin 2020/13**

(21) Application number: **15762711.8**

(22) Date of filing: **15.07.2015**

(51) Int Cl.:
**G16H 50/20** (2018.01)    **G16H 50/30** (2018.01)

(86) International application number:
**PCT/IB2015/055361**

(87) International publication number:
**WO 2016/097886 (23.06.2016 Gazette 2016/25)**

(54) **DIFFERENTIAL MEDICAL DIAGNOSIS APPARATUS ADAPTED IN ORDER TO DETERMINE AN OPTIMAL SEQUENCE OF DIAGNOSTIC TESTS FOR IDENTIFYING A PATHOLOGY BY ADOPTING DIAGNOSTIC APPROPRIATENESS CRITERIA**

DIFFERENZIALDIAGNOSTISCHE MEDIZINISCHE VORRICHTUNG ZUR BESTIMMUNG EINER OPTIMALEN SEQUENZ VON DIAGNOSTISCHEN TESTS ZUR IDENTIFIZIERUNG EINER PATHOLOGIE ANHAND DIAGNOSTISCHER RELEVANZKRITERIEN

APPAREIL DE DIAGNOSTIC MÉDICAL DIFFÉRENTIEL CONÇU POUR DÉTERMINER UNE SÉQUENCE OPTIMALE DE TESTS DE DIAGNOSTIC POUR IDENTIFIER UNE PATHOLOGIE EN ADOPTANT DES CRITÈRES DE PERTINENCE DE DIAGNOSTIC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **16.12.2014   IT MI20142149**

(43) Date of publication of application:
**25.10.2017  Bulletin 2017/43**

(73) Proprietor: **L.I.A. Di Giuseppe Capasso**
**09134 Cagliari (IT)**

(72) Inventors:
• **CONTU, Licinio**
**I-09100 Cagliari (IT)**
• **MASIA, Alessandra**
**I-09131 Cagliari (IT)**

• **CHISTOLINI, Pietro**
**I-00183 Roma (IT)**
• **FRUSTAGLI, Gianluca**
**I-00132 Roma (IT)**
• **RUSPANTINI, Irene**
**I-00154 Roma (IT)**
• **SAIS, Mattia**
**I-09013 Carbonia (CI) (IT)**

(74) Representative: **Tiburzi, Andrea et al**
**Barzanò & Zanardo Roma S.p.A.**
**Via Piemonte 26**
**00187 Roma (IT)**

(56) References cited:
**WO-A2-2008/031082     US-A1- 2010 229 044**
**US-A1- 2013 185 093     US-B1- 7 076 437**
**US-B2- 7 958 407**

**Description**

Field of the invention

The present invention relates to the field of medical diagnosis apparatuses, more specifically to a differential diagnosis apparatus adapted to determine the sequence of diagnostic tests required for identifying a pathology, by optimizing diagnostic appropriateness indexes.

Background art

Medical diagnosis is the result of a series of empirical probability evaluations based on the physician's professional experience, skills, clinical reasoning capability, intuition, and knowledge of the persons, family groups and population with whom he/she operates, as concerns the frequency of the various pathologies, their possible familiar aggregation, the people's social conditions and life style, and the environmental factors involved.

Based on all this, on the outcome of the interview with the patient, on the collected anamnesis data, on the symptoms complained of by the patient, and on the clinical signs detected through physical examination, the physician may already be able to formulate, in a small number of cases, a certain or highly probable diagnosis.

In most cases, the physician will only be able to formulate one or more diagnostic hypotheses to be verified by means of a number of laboratory and instrumental diagnostic tests. This process is defined as differential diagnosis.

The key point of the process is that, although a diagnosis may be correct, it may not necessarily be appropriate (for example, if the diagnosis has been formulated by using redundant diagnostic tests, i.e. tests which are unnecessary, unjustifiably expensive, or uselessly risky for the patient).

The diagnostic tests available today in medical practice are numerous and continually evolving, and the physician's choice of the tests to be made for a specific case can be very complex. It is apparent that the prescription of one or more tests can become particularly critical for general medicine physicians (GMP) who have to work transversally in different medical fields and who are often the patient's first contact.

The main risks associated with test selection are essentially two: 1) overuse of diagnostic tests, resulting in unjustified higher costs for the National Sanitary System (NSS) and negative effects on the patient's health, and 2) underuse of diagnostic tests, due to limited knowledge of an excessively broad offer, which may lead to unsuccessful diagnosis.

This situation has led several national and international medical/sanitary organizations, in recent years, to identify criteria to ensure performance quality (prescription of diagnostic tests and pharmacological treatments) and cost limitation, especially on the basis of outcome evaluations (evidence-based medicine) indicating the degree of effectiveness of a performance with beneficial effects on the patient. In this context, it is worth mentioning the study carried out by the Committee on the Quality of Health Care of the National Academy of Science in the United States (in Crossing the quality chasm: a new health system for the 21st century. Washington: National Academy Press; 2001), which identifies safety, effectiveness, timeliness, efficiency, equity and patient centrality as the targets of the National System of the 21st century.

It is in this context that the concept of diagnostic appropriateness has recently grown and become increasingly widespread in the medical field.

The appropriateness of a diagnostic test for a given pathology is not only determined by its accuracy, which can be expressed in terms of sensitivity and specificity, but also by several other factors: costs, access and outcome wait times, safety and tolerability for the patient, as described, for example, in: Ferrante di Ruffano L., Hyde C.J., McCaffery K.J. Bossuyt P.M.M. and Deeks J.J., "Assessing the value of diagnostic tests: a framework for designing and evaluating trials", British Medical Journal. 2012; 344-352.

The appropriateness concept extends to the entire diagnostic path. In fact, the physician's goal is to identify the disease that is most likely affecting the patient by starting from the collection of anamnesis data, symptoms and signs, and then by selecting the tests necessary for making the differential diagnosis, along a diagnostic path that is accurate, effective, fast, safe and economical, i.e. appropriate.

In this regard, it is apparent that the physician's capabilities would be improved if he/she could choose from a number of appropriate diagnostic paths and could know *a priori* how much the prescription of one or more tests would effectively refine the differential diagnosis in terms of costs and benefits for the patient and for the NSS.

In general, the automatic and computerized systems used as a support for clinical diagnoses rely on a general knowledge base (epidemiological data, interactions between drugs, guidelines, etc.), to be integrated with the patient's specific data (personal information, anamnesis, symptoms, signs). The data are then processed in order to formulate diagnostic hypotheses through the use of information theories and technologies (simulations, bioinformatics algorithms, statistical procedures, artificial intelligence systems).

Several attempts have been made in the past to develop software, or more generally ICT systems, which could help physicians make diagnostic decisions. Initially they were expert systems with structured databases developed within hospital structures, which were used as large reference systems. Actual diagnosis support systems, in some cases indicating the likelihood of the diagnostic hypothesis, have been created mainly for individual medical branches, where the theoretical and technical diagnostic problem remains at a low complexity level. Some systems use rules of inference (such as if, then) instead of probabilistic methods or heuristic algorithms.

More recently, diagnosis support systems have been developed which utilize a non-structured knowledge base and complex inference algorithms, e.g. as described in the article by Wagholikar, K. B.; Sundararajan, V. & Deshpande, A. W. "Modeling Paradigms for Medical Diagnostic Decision Support: A Survey and Future Directions", J. Medical Systems. 2012; 36 (5): 3029-49, or in the article by El-Kareh R., Hasan O., Schiff G.D. Use of health information technology to reduce diagnostic errors. BMJ Quality & Safety. 2013; 22(Suppl 2):ii40-ii51.

The following will briefly describe, by way of example, the most clinically interesting diagnosis support systems known in the art, which are dedicated to a wide range of diseases.

A first diagnosis support system, known by the acronym **DXplain**, is described in the article by Barnett G.O., Cimino J.J., Hupp J.A. Hoffer E.P., "DXPlain - An evolving diagnostic decision-support system", JAMA, 1987; 258(1): pp. 67-74. This is a clinical decision-support system that provides a list of possible diagnoses starting from a given set of signs, symptoms, epidemiological data and diagnostic tests (patient data), through the use of the Bayesian logic. The ranking of each pathology is defined by how close the set of patient data gets to the set of characteristic clinical manifestations of that specific disease, through the use of "pattern-matching" techniques. Each disease/patient-datum association is described by two numbers: 1) the frequency, organized on seven levels, at which the specific patient datum occurs in the disease (related to the concept of sensitivity) and 2) the evocative power, expressed on eight levels, of a patient datum, i.e. the force with which its presence confirms the disease (analogous to the positive predictive value of a). In addition, each patient datum has a disease-independent importance value expressed on five levels, indicating its diffusion in ill individuals and how rarely it is observed in healthy individuals. Finally, intrinsic values are associated with each disease, one for prevalence and one for importance (meaning the negative impact it would have if it were excluded from the list).

Upon request, the system provides an explanation of the reason why each one of the diseases must be taken into consideration, recommends further clinical information that needs to be acquired for clarifying the differential diagnosis, indicates which clinical manifestations are atypical for each disease, and provides ten bibliographic references for each disease. This system is probably the most advanced and complete first-generation system.

A system called **Medical diagnosis system** (by G. Fiore) is also known, which consists of differential diagnosis software for the Windows platform on PCs and mobile devices. This is a first-generation diagnosis support system similar to DXplain, wherein an empirical value (from 1 to 10) is associated with each symptom according to the frequency of the symptom in the different pathologies (it is more important if it is present in many diseases) and to the importance with which said symptom appears in the different pathologies. The user can set a minimum percentage threshold for considering a diagnostic hypothesis, and can make customized associations between symptoms and diagnoses. The system can be used for consultation purposes via an advanced search function by symptoms (such as: begins with, contains, and/or), which can be presented either in alphabetical order or grouped by organ or type (laboratory tests, objective test). The system then provides a list of possible diagnoses in alphabetical order. Finally, the user may actively choose to obtain an estimation of the probability associated with the diagnostic hypotheses for a differential diagnosis.

Systems have been recently proposed which allow fast and easy interrogation of a non-structured knowledge base made up of a wide range of clinical, diagnostic, scientific and epidemiological information useful to the physician during his/her working activity. In this context, two main systems can be identified: ISABEL (Isabel Healthcare Inc.) and WATSON (IBM).

The **Isabel** system, described in the article by Ramnarayan P. Tomlinson A, Rao A., Coren M., Winrow A., Britto J. ISABEL "A web-based differential diagnostic aid for paediatrics: results from an initial performance evaluation", Archives of Disease in Childhood, 2003;88:408-413, is a web-based diagnostic check list that allows manual entry of patient data (signs and symptoms, laboratory and instrumental tests, demographic data) and integration with different American standards of the Electronic Medical Handbook (EMB). Isabel uses a search engine based on natural language processing in order to identify matches between patient data entered as text, e.g. notes in the EMB, and similar terms in the semi-coded diagnostic dataset. Diagnostic hypotheses are sorted according to the force of their correspondence with the entered data. In addition to different diagnostic hypotheses, it also proposes possible therapies based on known scientific literature and protocols. The algorithms that allow the extraction of diagnostic hypotheses are proprietary and are based on a probability database.

The **Watson** system, described in patent application WO2012/122198-A1, is a massively parallel computer made up of 90 servers, 2,880 processors (cores) and 16 Terabytes of RAM, which can acquire and interpret a voice input (the question), process information at a speed of 500 Gigabytes per second, and give an answer through voice synthesis within three seconds at the latest. In recent years, IBM has steered towards medical applications, and is adapting the Watson system to create an automatic diagnosis system. The approach remains the same as the one already described. It is still a "QA machine" (question answering machine) which, in reply to a specific question (the clinical picture of a generic patient: signs, symptoms, etc.), outputs a list of most likely pathologies starting from a textual medical knowledge base stored in the memory (specialistic texts, manuals, scientific articles, guidelines, etc.). The first experimental applications have concerned the field of medical didactics and the specialistic field of pulmonary oncology.

Notwithstanding numerous attempts to develop diagnosis support systems, many of which have proved to be valid, none of such systems has yet come into current use in medical practice.

The criticalities of the systems known in the art, such as, for example, those described in the article by Kawamoto k., Houlihan C.A., Balas E.A., Lobach D.F. "Improving clinical practice using clinical decision support systems: a systematic review of trials to identify features critical to success", BMJ. 2005; pp. 330-337, and also described in Wagholikar et al, op. cit., include the following:

> **1)** the actual correctness of the proposed diagnosis, which can be evaluated in relation to the outcome for the patient;
> **2)** the need for a system that is truly integrated with the physician's current activity, i.e. available at the moment and in the place of the medical examination;
> **3)** the need for a system that can be easily and quickly consulted without requiring long training;
> **4)** the need for a system that offers recommendations as opposed to evaluations.

The first critical point of the known diagnosis support systems lies in the implementation of the physician's diagnostic reasoning model, which cannot consist merely of a system of empirical rules associating patient data with diseases, as in first-generation systems, or an automatic system of the "question & answer" type.

In brief, the criteria adopted by Watson, and also by Isabel and by the most important devices known in the art, are based on the development of a sort of refined search engine (like those of Google or Yahoo) which:

> **1.** has access to a huge amount of documentation (in this case, medical documentation) encoded in natural language (mostly English);
> **2.** uses natural language analysis algorithms, and
> **3.** evaluates all this documentation starting from the specific question asked to the device, assigning a "suitable" score to each document;
> **4.** uses an inferential engine (e.g. a Bayesian network) to output the list of the most likely diagnoses.

This kind of architecture, however, poses some problems in terms of accuracy. In fact, if on the one hand the use of a non-structured textual knowledge base is undoubtedly helpful because it offers an almost unlimited source of information, on the other hand it gives rise to problems in terms of extraction of useful and properly interpreted information. As a matter of fact, the typical problems associated with natural language processing become critical in the medical field, as described, for example, in the article by Nadkarni P.M., Ohno-Machado L., Chapman W.W. "Natural language processing: an introduction", Journal of the American Medical Informatics Association, 2011; 18: 544-551, because of the extremely synthetic language, abbreviations, compound words and synonyms in use. Also critical are the segmentation of the text in significant groups and the categorization of specific words. Finally, the accuracy of the answer is related to the amount of text entered in the specific query based on natural language.

Currently, in fact, the Watson system is being tested in a specific medical field, and the Isabel system is resorting to a mixed, i.e. structured and non-structured, knowledge base. Both of these strategies allow reducing the complexity of the problem and obtaining more accurate diagnoses.

Although the Watson and Isabel systems can provide an answer to a single interrogation in a few seconds, the time necessary for formulating a diagnostic hypothesis is considerably longer because multiple queries are necessary, and the data entry process in successive steps does not appear to be easy.

The second critical point of the known systems lies in the fact that they do not take into account appropriateness criteria in the different steps of the diagnostic process. In this context, the meaning of appropriateness is known to those skilled in the art. To our knowledge, the apparatuses available today only consider the effectiveness of a treatment on the basis of evidence-based medicine, neglecting other fundamental aspects of diagnostic appropriateness, including timeliness, cost limitation, safety and patient centrality, in the various stages of the diagnostic path. One highly critical step is certainly the prescription of diagnostic tests, the variety and numerosity of which poses serious problems in terms of over/under-diagnosis, as already mentioned above, i.e. inappropriateness problems adversely affecting the patient's health and the costs incurred by the NSS.

The current systems do not take into account the fact that a physician needs to obtain support throughout the diagnostic path, which includes the prescription of appropriate diagnostic tests. The Watson system suggests to the physician what information could be useful to improve diagnosis confidence, without however recommending a diagnostic path. The presentation of a sorted list of probable diseases based on the patient's clinical picture may not be sufficient for the physician to be able to formulate an appropriate diagnosis. Most likely, diagnostic tests will be required for confirming and excluding some diseases. If the entire path is not taken into consideration, there will be a risk of remaining within the precincts of subjective and empirical evaluations, while also systematically resorting to numerous, expensive and partly useless diagnostic tests and neglecting other tests which may be more appropriate, though less known.

It is therefore necessary to find a medical diagnosis support system which can empower the physician's knowledge by integrating it with the knowledge of scientific literature and national and international guidelines available in a dynamic database, and which can compensate for the tendency towards over/under-diagnosis through a guided test selection made according to diagnostic appropriateness criteria, while also reducing the hesitation that is inherent in the physician's human judgement.

The relevant prior art includes also the patent application US 2013/185093 A1 and the patent US 7076437 B1.

Summary of the invention

The present invention aims at providing a medical diagnosis apparatus adapted to determine the optimal sequence of diagnostic tests for identifying a pathology according to appropriateness criteria, which can overcome all of the above-mentioned problems by formulating complete diagnostic test paths on the basis of appropriateness indexes, and which can be easily and quickly consulted during a medical examination.

It is therefore the object of the present invention to provide a medical diagnosis apparatus useful to the physician when making a differential diagnosis, which allows a guided selection of diagnostic tests for determining appropriate diagnostic paths.

In the following, the term **diagnostic tests** will refer to all coded procedures aimed at formulating a diagnosis by confirming or excluding a pathology.

By way of non-limiting example, this definition includes laboratory, instrumental and clinical tests as well as questionnaires (standard/coded questions) for the physician about signs and symptoms not detected and/or or not reported by the patient.

The sequence of diagnostic tests determined by the apparatus of the invention is defined as **diagnostic path.** The apparatus of the invention determines an appropriate diagnostic path for formulating a diagnosis on the basis of the determination of a global appropriateness index (of the entire diagnostic path) obtained from the numerical appropriateness indexes of each diagnostic test.

The object of the present invention is, therefore, to provide a medical diagnosis support device by processing an appropriate path of diagnostic tests.

The device of the invention can thus "support" the physician by recommending diagnostic paths optimized in terms of appropriateness (e.g. the least expensive and/or fastest path). The apparatus of the present invention is made up of **modules**, which are essentially created by means of software implemented in storage devices readable and executable by a system of electronic processors equipped with input/output devices.

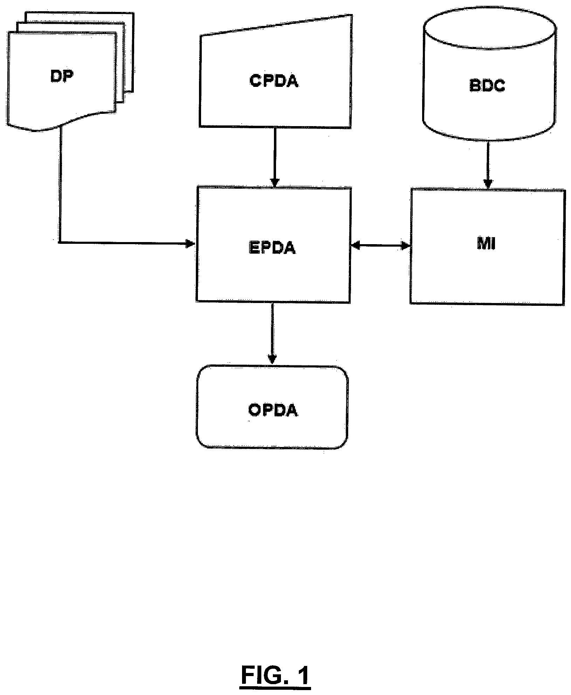

Such modules are represented in the block diagram of Figure 1, wherein two main blocks can be distinguished. An **MI** module operating as an "inferential engine", and an **ePDA** module dedicated to "processing the appropriate diagnostic path" .

The **ePDA** module receives, as input from the **DP** module, data pertaining to the patient information and receives from the **cPDA** module data pertaining to the specific configuration of the system variables to be used for processing the appropriate diagnostic path (**PDA**). Starting from these data, the inferential engine **MI** interrogates a knowledge base (**BDC**), and processes and provides to the **ePDA** module the information necessary for processing the diagnostic paths and generating, as a result, the **diagnostic path** to be used by the physician.

More in particular, still with reference to Figure 1:
In the **DP** module (*PATIENT DATA*) the patient's data are managed both in an interactive manner and from an electronic archive (e.g. patients database, electronic medical handbook, etc.), whether locally or remotely.

The *cPDA* module (**PDA** CONFIGURATION) stores the configuration of the system variables, including also the configuration of the appropriateness parameters (such as, without limitation, cost, time, safety and tolerability of the test for the patient), in order to compute the global appropriateness index to be optimized in the diagnostic path.

The **BDC** module (KNOWLEDGE BASE) comprises a relational database containing all data pertaining to pathologies, signs, symptoms, drugs, reference values of clinical analyses, e.g. obtained, without limitation whatsoever, from epidemiological databanks, guidelines and scientific literature, databanks of clinical cases, patient data history archive, and diagnostic path processing steps carried out by the **MI** for each patient. It also contains the values of the appropriateness parameters of tests that may be prescribed in order to complete the diagnostic path, such as, by way of non-limiting example, sensitivity, specificity, safety, cost and wait times necessary for the computation of the most appropriate paths. The relational database is populated by using a *per se* known methodology.

The **MI** module (INFERENTIAL ENGINE) uses an algorithm in order to compute, by carrying out an inferential procedure starting from the patient data, the values of the probabilities for each diagnostic hypothesis. The module also proposes, for each hypothesis, one or more diagnostic tests and computes how much a particular test sequence (diagnostic path) will affect the likelihood of the different hypotheses, associating with each path a respective appropriateness index.

The inferential procedure adopted in the **MI** module is preferably, but not exclusively, based on one of the algorithms known in the scientific literature (e.g. see the aforementioned review by Wagholikar et al.). For example, the algorithms used for diagnostic inference may be based on "*fuzzy logic*" relationships between symptoms and diseases, artificial neural network models, Bayesian networks combining symbolic reasoning with the Bayesian statistical approach, and *"support vector machines"*, i.e. a supervised learning method for non-linear classification and regression. The following will describe in detail an inferential engine based on the Bayesian statistical approach, as one of the possible algorithms that may be implemented.

The **oPDA** module (Output **PDA**) displays a list of diagnostic hypotheses (i.e. pathologies) which are considered to be most likely, and for each one of them it processes and shows the possible diagnostic paths with their respective appropriateness indexes.

The present invention is characterized by the following aspects:

1. The apparatus determines a list of diagnostic hypotheses by starting from the patient data and the knowledge base, and quantifies the probability associated therewith (pre-test probability).
2. The apparatus determines, for each diagnostic hypothesis, appropriate diagnostic paths by using information retrieved from consolidated and updated databases, medical and epidemiological records coming from international, national and regional institutes.
3. The apparatus associates an appropriateness index with each test and each diagnostic path in accordance with national and international guidelines.
4. The apparatus predicts the impact (post-test probability) of a diagnostic test or, more in general, of a diagnostic path by starting from the previously determined pre-test probability, before the test is executed.

One important aspect of the invention is that it provides, thanks to the support defined along the entire diagnostic path, faster, less expensive and more certain, i.e. more **appropriate** (effective, efficient, safe), diagnoses in the most complex cases that a generic physician or specialist may have to face.

It is a particular object of the present invention to provide a differential diagnosis apparatus adapted for medical applications in order to determine an optimal sequence of diagnostic tests for identifying a pathology by adopting diagnostic appropriateness criteria, as set out in the claims, which are an integral part of the present description.

### Brief description of the drawings

Further objects and advantages of the present invention will become apparent from the following detailed description of a preferred embodiment (and variants) thereof referring to the annexed drawings, which are only supplied by way of non-limiting example, wherein:

Figure 1 shows a functional block diagram of the medical diagnosis apparatus according to the present invention;
Figure 2 is a diagram showing the functional interrelation among blocks of the database system of the apparatus;
Figure 3 is a diagram showing the interaction among the interactive menus of the apparatus.

### Detailed description of some embodiments of the invention

The following will describe one example of embodiment of the apparatus according to the invention, with reference to the flow chart of Fig. 1.

At the centre of said diagram there is the block for processing the appropriate diagnostic path **ePDA**, which, given the system configuration **cPDA**, produces the output information **oPDA**. These blocks realize a vertical information flow **PDA**, which makes also use of the connection to the system database **DB**, divided into two further blocks: the patient data database **DP** and the knowledge base database **BDC**. The data coming from the latter are entered into the vertical information flow **PDA** through the inferential engine block **MI.** The two sections (**BDC** and **DP**) that make up the database **DB** may also be physically located on distinct hardware systems. The database **DP** may consist, for example, of the physician's private patients archive, interfaced to the diagnosis support system.

As aforesaid, the set of diagnostic tests that the physician will finally prescribe is called **diagnostic path.** A diagnostic test or path can be defined as appropriate for formulating a diagnosis in accordance with a general principle of cost/benefit ratio minimization, based on the determination of different **appropriateness parameters**, by associating a numeric value with each one of them.

Some non-limiting examples of **appropriateness parameters** are as follows:
The first one is undoubtedly the **Effectiveness (E)** of a test in confirming or excluding a specific pathology. The effectiveness of a test indicates how much the knowledge for a pathology progresses or regresses on the basis of the outcome, evaluated in terms of variation of the probability of a diagnostic hypothesis before (pre-test probability) and after (post-test probability) the execution of the test.

In order to determine if the execution of a test is certainly appropriate, it is also necessary to evaluate its economical cost. The **Cost (C)** parameter refers to the cost incurred by the National Sanitary System, as defined by the National Health Care Range of Fees.

Also the **time (Te)** required for accessing the test is a parameter useful for defining the appropriateness of a test, in that the test will clearly have to be executed as quickly as possible depending on the severity of the suspected disease. The wait time data are those defined by the National Waiting-List Plan.

Other aspects that characterize test appropriateness are patient safety and tranquillity.

The **Risk (R)** parameter takes into account the *Intrinsic risk* of the procedure itself and the *Relative risk* dependent on the patient's pathological condition.

Patient tolerability is important to ensure that the prescription will be followed and the path will actually be completed. The **Tolerability (To)** parameter is defined in a subjective way by means of, for example, a questionnaire to be filled by the patient.

All appropriateness parameters can be suitably associated with numerical values.

## THE DATABASE

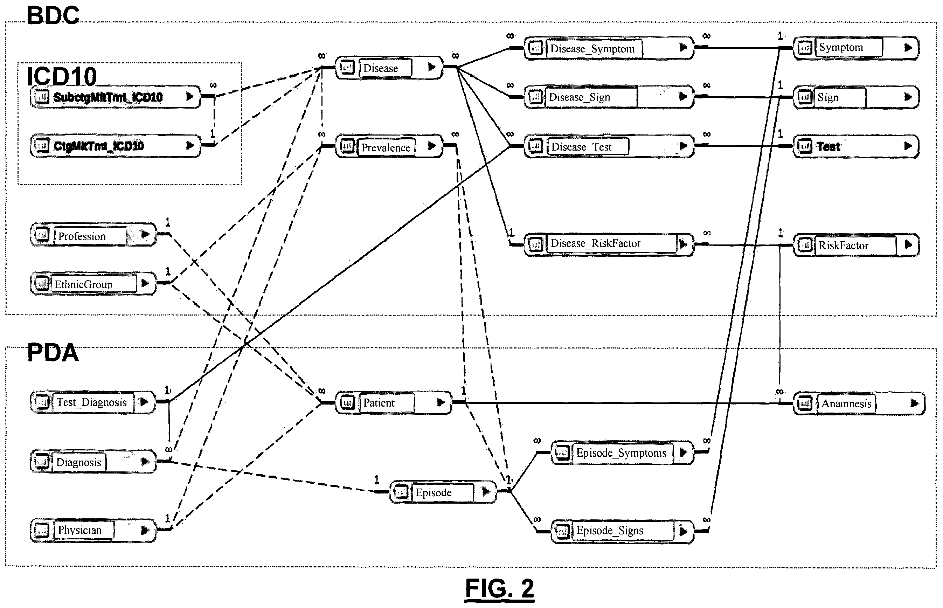

The information contained in the database **DB**, in the form of record tables interconnected by a network of relations is essentially of two kinds, as generally indicated in Figure 1, and as will be described in detail below with reference to Figure 2.

> **1. BDC**; an articulated set of preloaded data structures constituting the "**knowledge base**", on which all logical choices and processing steps of the application are based;
>
> **2. DP**; specific data managed by physician users, pertaining to signs, symptoms and anamneses, entered during medical examinations (**Patient data**). **DP** also contains the data relating to the processing results, in terms of diagnostic hypotheses and tests to be carried out (i.e. the data produced by the vertical processing flow of the appropriate diagnostic path **PDA**).

### Knowledge base BDC of the application.

The knowledge base is structured as a set of variously interconnected tables with preloaded and updateable contents, as shown in Fig. 2.

The **Disease** table plays a central role in the "knowledge base" of the application: it identifies the diseases known to the application as univocal combinations of categories and sub-categories according to the **ICD10** classification. In fact, the tables connected thereto, i.e. **CtgMltTmt_ICD10** (ICD10 disease and traumatism categories) and **SubctgMltTmt_ICD10** (ICD10 disease and traumatism sub-categories), contain the corresponding lists of the tenth revision of the international classification of diseases and related health problems as proposed by OMS (ICD10).

The **Prevalence** table, which shows the probability value for each disease, normalized by sex, ethnic group and age range of the patient, is connected to the Disease table.

The **Symptom**, **Sign** and **Test** tables contain, respectively, the lists of all observable symptoms and signs and the list of all executable medical tests (instrumental tests). These tables allow the application to interpret the information entered by the physician during the examinations, allowing the selection among the various options available.

The **Test** table also indicates, in addition to the test name and description, whether it is a dichotomous test (positive/negative - range: Dichotomous) or a continuous-range test (in which case it also states the unit of measure in use - range: Unit).

This is of fundamental importance for the computation algorithm, in that the chosen approach is to logically treat and reduce each continuous-range test to a series of "virtual" dichotomous tests by dividing the variability range of the possible results into an adequate number of sub-ranges (according to this approach, every value resulting from the execution of the test will fall within one of the possible sub-ranges, thus causing the corresponding dichotomous sub-test to be positive).

This table also comprises the numerical values of some appropriateness parameters associated with the test: cost, wait time, and intrinsic risk.

The **Disease_Symptom** table expresses the many-to-many relationship existing between the Symptom table and the Disease table (each disease may have multiple symptoms - or none - and each symptom may be common to multiple diseases), and includes, as attributes, the probability of the presence of the symptom in subjects suffering from the corresponding disease and the probability of the absence of the symptom in subjects not suffering from the corresponding disease.

Like the previous one, the **Disease_Sign** table expresses the many-to-many relationship between the Sign table and the Disease table.

Likewise, the **Disease_Test** table expresses the many-to-many relationship existing between the Test table (instrumental tests) and the Disease table (each disease may be found by one or more combined or alternative tests - or none - and each test may be useful for finding multiple diseases). In this case, the attributes are the sensitivity and specificity of the test with respect to a specific disease.

The **RiskFactor** contains a list of risk factors of various types that may predispose to or cause diseases.

A many-to-many relationships binds the risk factors to the Disease table, said relationship being implemented in the **Disease_RiskFactor** table.

The **Profession** and **EthnicGroup** tables contain a list of, respectively, known professions and ethnic groups which are relevant from a medical viewpoint. Both are used (via 1-to-many relationships) by the Patient table. The EthnicGroup table is also related to the Prevalence table.

**Tables and structures for the specific patient data DP.**

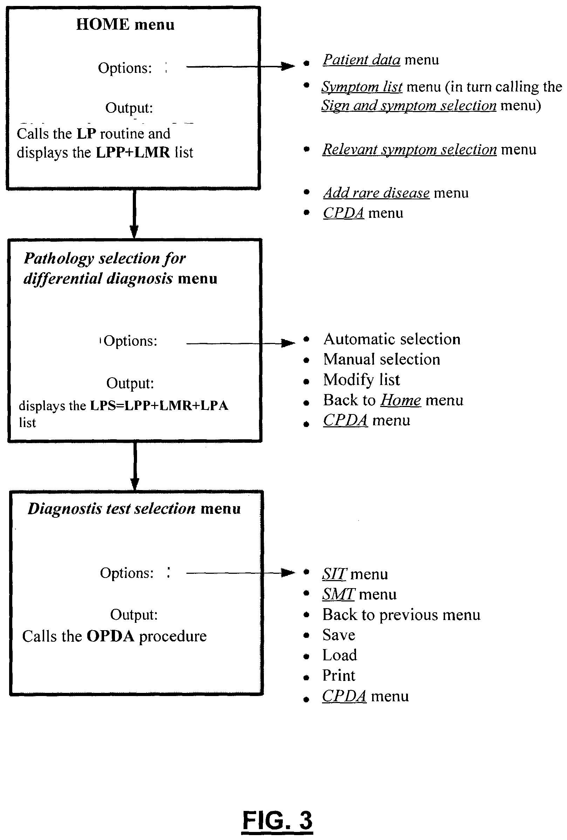

The specific patient data include a set of variously interconnected tables with preloaded and updateable contents, as shown in Fig. 3.

The application is of course designed for use by different physicians; therefore, the **Physician** table contains some personal information, and all the specific data pertaining to each patient, entered over time, will be stored into the database and related to the physician's identifier.

The **Patient** table contains basic information about each patient.

The Patient table is connected, via a 1-to-many relationship, to the **Episode** table. Each record in said table identifies an *episode* in the patient's clinical history, which begins from the first examination, during which the physician collects signs and symptoms connected to a certain disorder, and ends with the diagnosis, possibly after a number of instrumental tests. Should the patient subsequently return to the doctor's for another disorder (different from or similar to the previous one), the physician will open, by using the application, another clinical episode that will imply the creation of a new record in the Episode table, connected to the same patient.

The Episode table is related to the **Episode_Sign** and **Episode_Symptom** tables: during the examination, the physician will enter the symptoms reported by the patient. The two Episode_Sign and Episode_Symptom tables express, by populating them, two many-to-many relationships between Episode and Sign and between Episode and Symptom. Something similar occurs in the **Anamnesis** table. The Anamnesis table is connected to the Patient table. The patient's case history data are entered only once and may possibly be updated over time. Also the Anamnesis table expresses a many-to-many relationships between the Patient table and the RiskFactor table. The anamnesis data are collected by listing all risk factors to which the patient is exposed.

After all said information has been entered, the application is ready for the first processing step that will compute the first diagnostic hypotheses on a probabilistic base (pre-test probability) through the use of the algorithm implemented in the inferential engine MI. The resulting list of pre-test diagnostic hypotheses is then stored into the **Diagnosis** table. Then the post-test probabilities are computed by means of the inferential engine **MI**, which resorts to a series of queries that cross-reference the list of pre-test diagnostic hypotheses in Diagnosis with the Disease_Test and knowledge base Test tables, obtaining for each one of the hypothesized diseases all the associated instrumental clinical tests and the respective sensitivity and specificity values and parameters for the calculation of the appropriateness level of each test.

It is thus determined which tests, among those just determined, are most useful for gaining information for the final diagnosis.

These results are stored into the **Test_Diagnosis** table, wherein a series of tests are associated with each diagnostic hypothesis, with their respective values of the intrinsic appropriateness parameters (cost, wait time, intrinsic risk) and post-test probabilities.

The values of additional appropriateness parameters may then be entered into the same table, such as relative risk and tolerability of the test, which will be used in the optimization step, wherein a test appropriateness index will be computed in order to give the physician some more information useful for the final choice of the tests to be prescribed.

These same records may also subsequently receive the values of the actual results of the prescribed diagnostic tests actually carried out, for future use.

**INFERENTIAL ENGINE MI.**

This is the module that implements the algorithm for computing, starting from the patient data **DP** and, as previously described, from the set of preloaded data structures that make up the "**knowledge base**" BDC, the probability values for each diagnostic hypothesis. The module also proposes, for each hypothesis, one or more diagnostic tests,

and computes how much a particular test sequence will affect the likelihood of the different hypotheses, associating with each path a respective appropriateness index.

The inferential procedure may be based, for example, on any one of the algorithms cited in the scientific literature (the most common ones being those based on fuzzy logic relationships between symptoms and diseases, artificial neural networks, Bayesian networks, and support vector machines). The following detailed description will present, as one of the possible algorithms that may be implemented, an inferential engine based on a Bayesian approach known as NAIVE BAYES. More in general, the choice of the algorithm will not lead to different final diagnoses, but may affect the diagnostic path in terms of how quickly the final diagnosis will be arrived at. In other words, all algorithms, also because of the interactivity of the procedure, will lead to the same diagnosis, though they may require different times.

The algorithm selection will determine, case by case, the necessity of modifying the **DB** to include therein the data required for the execution of that specific algorithm.

At any rate, the **DB**, as described above, still contains the information necessary for implementing a NAIVE BAYES type algorithm.

Let us now consider, by way of example, the general case of a Bayesian network. Mathematically speaking, a Bayesian network is a directed acyclic graph wherein the nodes represent the variables, the arcs represent the relationships of statistical dependence between the variables and the local probability distribution of the leaf nodes with respect to the values of the parent nodes.

In general, every pathology, sign, symptom, anamnesis datum, and test is, without distinction, a statistical variable, and hence a node of our Bayesian network. With each directed arc that binds a generic node A to a node B, a conditional probability $P(B|A)$ must be associated; the knowledge of the probability values of all arcs in the network allows the formal computation of the probability for any one node, conditional upon the probability associated with any other set of data nodes. We will thus be formally able to compute the probabilities $Pre(M | \{DP\})$ and $Post(M | \{DP\} \cup \{T\})$, i.e. the pre-test and post-test probabilities of finding the pathology M, respectively conditional upon the patient dataset $\{DP\}$ and the union of $\{DP\}$ and the test set $\{T\}$.

To do so, we will have to add to the previously described **DB** the information about the network structure (a many-to-many table) and the value of the probability functions associated with each arc.

Therefore, as aforementioned, the choice of the algorithm implies additions to the database structure. In this respect, we will mention herein another type of implementation of the inferential engine MI.

Reference was made previously to, among others, those algorithms known as *support vector machines;* these represent a particular case of the so-called *machine learning* techniques. By way of example, such techniques can be implemented as follows: the probability of a pathology is represented as a non-linear function of the patient data (signs, symptoms, tests, etc.). When the latter are known, said probability can be easily computed. The difficulty lies in knowing the values of those parameters and coefficients of that function which are not known *a priori.* They may be predetermined by using automatic learning procedures typical of the machine learning techniques. Once determined, the parameter and coefficient values have to be entered into a supplementary many-to-many table of the database. What has been described by way of example in the last paragraph is applicable to any algorithm. In general, all preloaded data structures making up the "**knowledge base**", necessary for the **MI** to estimate the probability of a pathology, can be preliminarily obtained:

1. from epidemiological databanks, guidelines and scientific literature.
2. from clinical databases (e.g. electronic medical handbook).
3. from the history of appropriate diagnostic path processing steps carried out by the **MI** and stored in the **DB.**

If the data sources described at 2 and 3 above are used, well-known *supervised machine learning* techniques will be employed in order to "compute" those parameters which, once "preloaded" into the knowledge base, will allow the **MI** to process the **PDA** for each patient. It is also conceivable to use the same procedures for periodically updating and consolidating the knowledge base **BDC.**

As far as the vertical information flow **PDA** of Fig. 1 is concerned, it includes three successive functional steps (the user may nevertheless return to a previous step to review and change the previous settings):

1. **Appropriate diagnostic path configuration** (**cPDA** block in Fig. 1);
2. **Pathology selection for differential diagnosis** (**ePDA** block in Fig. 1);
3. **Diagnostic test selection** (**oPDA** block in Fig. 1).

**Appropriate diagnostic path configuration** (**cPDA** block in Fig. 1).

During the step of Appropriate diagnostic path configuration it is possible to display, select and possibly modify the system variables, i.e. the parameters that will be used in the next steps for processing and generating the diagnostic paths. Such parameters also include those necessary for computing the appropriateness indexes. During the next steps it will still be possible to return to this step in order to reconfigure, whether partially or totally, the whole apparatus.

**Pathology selection for differential diagnosis** (**ePDA** block in Fig. 1).

In this step, the apparatus connects to the patient database DP to acquire, in addition to the patient's personal data, also the anamnesis data necessary for the next processing steps. With the patient thus selected, a list of signs and symptoms observed and/or reported during the medical examination is associated.

This information allows generating a list of most likely pathologies (LPP), processed by the inferential engine MI. Said list is joined by a list of rare diseases (LMR), which is processed by entering also pathologies not included in LPP but present in the database **DB**, for which, however, one of the detected signs/symptoms is highly specific (or has high sensitivity).

The total list can then be interactively reviewed and updated by the physician user.

In particular, in order to verify that all relevant symptoms have been considered for the pathologies of interest, one or more pathologies can be selected among those included in the list for further investigating the correlated symptoms and verifying the presence of additional typical symptoms not yet detected/reported.

Then a list of pathologies for which diagnostic paths are to be estimated is interactively processed; from the list of probable pathologies LPP, those pathologies are selected which have a post-signs-symptoms probability higher than a predefined and modifiable threshold (in **cPDA**). To LPP and to the list of rare diseases, one can add the list of manually added pathologies LPA; the total list LPS is thus given by LPS = LPP + LMR + LPA.

Therefore, at this stage it is possible to modify and expand LPP:

- manually: any pathology can be added at any time.
- by changing the threshold for the selection of the most likely pathologies.
- by directly editing the list: any pathology can be selected/unselected from the final list

Once LPS has been consolidated, the next step can be carried out.

**Diagnostic test selection** (**oPDA** block in Fig. 1).

A set of tests (obtained by interrogating the DATABASE) corresponds to each pathology of the selected list LPS. The union of all these sets for all pathologies constitutes a TAMS (tests associated with selected diseases) set.

In general, for selecting the diagnostic tests to be executed, different modes or combinations thereof can be used:

**1.** Semi-automatic interactive selection of tests with high diagnostic effect. From the TAMS set of all relevant tests, only those having a sensitivity and/or specificity higher than an *effectiveness* threshold, the value of which can be changed, are selected. Then the post-test probability is computed for each pathology, assuming that every single diagnostic test will be executed. This identifies those tests which, if carried out, will cause the knowledge to progress or regress. The tests with a high diagnostic effect will be those which exceed a post-test probability threshold, also modifiable by the physician.

**2.** Manual test selection. For each pathology, the physician can display all the tests associated therewith and select one or more diagnostic tests at will. Thus, also a test not exceeding the effectiveness threshold can be selected. For each selected test, the system computes the post-test probability and the appropriateness.

**3.** If necessary, the physician may also select any test from the DB, regardless of its relevance. The tests selected with this option will be entered into the TAMS set.

If more than one diagnostic test is chosen, the apparatus will also compute the post-test probability of each diagnostic hypothesis, assuming that all tests will be carried out, and the appropriateness of the entire diagnostic path (i.e. the global appropriateness of all selected tests), in addition to displaying the post-test probability and the appropriateness of each test.

The above-mentioned procedure associates with each test its respective appropriateness index **IA**, which, by way of example, can be defined as:

$$IA = To \: / \: (C * Te * R)$$

where Te is the test access wait time index; C is the test cost index; R is the index of the maximum between the intrinsic risk and the relative risk of the test; To is the test tolerability index.

A global appropriateness index ($IA_G$) is also determined for all the selected diagnostic tests, which is also computed by using the above formula, i.e.:

$$IA_G = To_G / (C_G * Te_G * R_G)$$

where: $To_G$ is the minimum among all tolerability indexes of all tests in the sequence, $C_G$ is the sum of the single costs of each test in the sequence, $Te_G$ is the maximum among all wait times, $R_G$ is the maximum among all risk indexes of the sequence.

In summary, the output consists of the following data, preferably shown on a display (in the **oPDA** block in Fig. 1) for each diagnostic hypothesis:

- the pre-test probability,
- the list of all tests sorted by appropriateness, with their respective post-test probability and appropriateness index,
- the global post-test probability and appropriateness index of the entire diagnostic path.

Different diagnostic paths can be constructed, even by adding and removing individual tests from the selection, and then the post-test probabilities of the pathologies and the appropriateness indexes of the paths can be re-calculated.

It is therefore possible to define an alternative path (by selecting a different set of tests): the apparatus will keep in memory the previous path with its appropriateness values, so that different paths can be compared.

**Detailed description of an example of embodiment.**

The present invention is preferably implemented through software modules stored on storage devices readable and executable by a computer (e.g. server, desktop, workstation, notebook, tablet, smartphone, etc.) equipped with mass storage and input/output devices (see diagram of Fig. 1).

For example, the software modules are implemented on (physical or virtual) computation clusters, and can be executed remotely and implemented as Apps for mobile devices (tablets, smartphones, notebook PCs).

The implementation of the hardware is within the grasp of a man skilled in the art; therefore, no further details are necessary.

Such software modules will use the information stored in the knowledge base BDC and in the patient data archive DP; both databases have been described above in the section entitled "THE DATABASE".

All software modules and the whole database are developed by resorting to a Ruby on Rails framework used as a RAD environment and equipped with a series of opensource (gems) additional modules made available by the community of developers that supports this environment. In most cases, in this kind of implementation solution all non-volatile data and information necessary for the operation of the application are managed by using a relational database. In this case, a MySQL database server is used, connected to the application via a local socket. The man skilled in the art will be able to implement the software part by relying on his basic knowledge and on the present description.

With reference to Figure 3, the output device will display a sequence of interactive menus, the most important ones being three:

- *Home menu*
- *Pathology selection for differential diagnosis menu*
- *Diagnostic test selection menu*

Said menus will call the following menus and the two main procedures:

- *CPDA (Appropriate diagnostic path configuration)*
- *Patient data*
- *Sign and symptom selection*
- *Symptom list*
- *Selection of relevant symptoms*
- *Add rare disease*
- *SIT (Interactive test selection)*
- *SMT (Manual test selection)*

Procedures:

- **LP** - calculates the sorted list of the most likely pathologies
- **OPDA** - processes the output of the appropriate diagnostic path

**Description of the operation of the individual menus and procedures.**

(in association with each menu option, the following will provide a description of the option and the list of the activities, in pseudo code, that will be executed when the option is activated).

*HOME* **MENU**

In this menu it is possible to call the sub-menus in which one can configure the system (**CPDA** menu), identify the patient and his/her clinical data, select signs, symptoms and anamnesis data (Patient data menu). This information allows processing a list of most likely pathologies (LPP) and displaying it in this menu. The list of rare diseases (LMR) is also associated with this list. The total list can then be reviewed and updated by using the menu options listed below.

**"Ignored symptoms by pathology" option:** for verifying that all relevant symptoms have been considered for the pathologies of interest.

A pathology is selected from those included in the list for further investigating the related symptoms and looking for any additional typical symptoms that have not yet been detected/reported.

If there are any, new symptoms can then be entered that were not entered during the first investigation step. The list of the most likely pathologies will be updated accordingly.

**"Ignored symptoms by importance" option:** for refining the differential diagnosis among the most likely hypotheses.

For the first diagnostic hypotheses in the list, the number of which is set by the NuSePa variable (e.g. 5 by default), all symptoms not reported during the first investigation step are extracted. Only the first 10 symptoms are displayed (default value of the SiPr variable) with the highest absolute LR (likelihood ratio) (maximum LR of a symptom among the most likely pathologies).

If there are any, the new symptoms not entered during the first investigation step can then be entered. The list of the most likely pathologies will be updated accordingly.

**"Significant symptoms already included" option:** for verifying that a pathology that is not very likely (rare disease) has not been neglected although a highly relevant symptom was detected.

Among all symptoms reported during the first investigation step, the system will display the first 10 symptoms (default value of the SiPiuP variable) having the highest LR and the pathology for which such value is highest.

The low-probability pathology can then be entered into the list of diseases to be taken into account in the subsequent menus.

### Home menu (procedure).

available options:

**Appropriate diagnostic path configuration**

opens the *CPDA* menu

**Patient data**

opens the *Patient data* menu

the displayed output is as follows:

IF ( "the patient data and symptoms have not been entered yet")

THEN PRINT ("enter patient data, signs and symptoms")

ELSE  call the **LP** procedure

displays the LPP + LMR list

turns on supplementary options in the *Home* menu

supplementary options:

**Ignored symptoms by pathology**

when a pathology is selected from the list (by clicking on it),

opens the menu *Symptom list*

**Ignored symptoms by importance**

opens the *Selection of relevant symptoms* menu

**Significant symptoms already included**

opens the *Add rare disease* menu

**Next**

opens the

*Pathology selection for differential diagnosis* menu

### *PATHOLOGY SELECTION FOR DIFFERENTIAL DIAGNOSIS* MENU

In this menu, the list of pathologies for which diagnostic paths need to be estimated is processed.

The three main options are:

**Automatic selection**: the selected pathologies are those which have a post-signs-symptoms probability higher than a 40% threshold (default value of the SoSePa_inf variable), but any "rare diseases" possibly selected in the previous menu will be kept in the list.

**Manual selection**: the physician can add any pathology at any time.

**Modify list**: the physician can select/unselect any pathology in the final list
*Pathology selection for differential diagnosis* **menu (procedure).**

available options:

**Automatic selection**

updates the LPP by considering only the pathologies having a probability value higher than or equal to that of the SoSePa_inf variable

**Manual selection**

the physician enters a pathology that he/she considered to be relevant, the latter is entered into the LPA list (pathologies manually added by the physician) along with the respective pre-test probability value.

**Modify list**

displays the entire list of selected pathologies LPS; the pathologies can be unselected/selected by clicking on them; the selected ones are displayed in bold.

**Configuration**

opens the *CPDA* menu

**back**

returns to the *Home* menu

**next**

*opens the Diagnostic test selection* menu; the output displays the list of selected pathologies (LPS), consisting of the sum of the LPP list (updated with the value of the SoSePa_inf variable) + LMR + the

list of pathologies manually added by the physician (LPA), hence

$$LPS = LPP + LMR + LPA$$

### *DIAGNOSTIC TEST SELECTION*

A set of tests (obtained by interrogating the DATABASE) corresponds to each pathology of the selected list LPS. The union of all these sets constitutes the TAMS (tests associated with selected diseases) set. For the preliminary construction of the TAMS set, not all pathologies of LPS are taken into account, since those tests are excluded which are associated with pathologies having a pre-test probability already higher than 70% (default value of the SoSePa_sup variable). This choice is due to the fact that the pathologies with high pre-test probability already provide a strong diagnostic indication without requiring other tests that would add less information than obtainable with tests more specific for the pathologies within the diagnostic uncertainty interval (defined by the SoSePa_sup and SoSePa_inf variables, having respective default values of 70% and 40%).

In general, in order to select the diagnostic tests to be executed, the physician can adopt different modes or combinations thereof:

**Semi-automatic interactive selection of tests with high diagnostic effect:**

From the TAMS set of all relevant tests, only those having an LR above an *effectiveness* threshold (SoEf), the value of which can be set by the physician in the **PDA Configuration** menu (by default SoEf = 2), are further selected.

The post-test probability is computed for each pathology, assuming that every single diagnostic test will be executed. This identifies those tests which, if carried out, will cause the knowledge to progress or regress. The tests with high diagnostic effect will be those exceeding a post-test probability threshold (by default 70%, and anyway lower than or equal to SoSePa_sup).

**Manual test selection**

For each pathology, the physician can display all the tests associated therewith and sorted by likelihood ratio, and select at will one or more diagnostic tests. Thus, also a test not exceeding the effectiveness threshold can be selected. For each selected test, the system computes the post-test probability and the appropriateness.

If necessary, the physician may also select any test from the *Diagnostic tests* list, regardless of its relevance for the diagnostic hypotheses and its effectiveness for the differential analysis. The tests selected with this option will be added to the TAMS set.

If more than one diagnostic test are chosen, the system will also compute the post-test probability, assuming that all tests will be carried out, and the appropriateness of the entire diagnostic path, in addition to displaying the post-test probability and appropriateness of each test.

The physician can thus build different diagnostic paths at will by adding and removing individual tests from the selection and by recalculating the post-test probability of the pathologies and the appropriateness of the paths.

### *Diagnostic test selection* menu (procedure).

available options:

**Semi-automatic interactive selection of tests with high diagnostic effect**
opens the *SIT* menu
**Manual test selection**

when a pathology is selected by clicking on it,
opens the *SMT* menu

**Configuration**
opens the **CPDA** menu
**Back**
opens the *Pathology selection for differential diagnosis* menu
**Save**
Saves the diagnostic path table (under a name)
**Load**

Loads a previously saved diagnostic path table
**Print**
Prints the diagnostic path table and displays the output of the **OPDA** (Appropriate diagnostic path output) procedure. In detail, for each pathology it displays the pre-test probability and then lists, sorted by appropriateness, all tests with their post-test probability and appropriateness, and, finally, the probability and appropriateness of the entire diagnostic path.

## *PATIENT DATA* MENU

From this menu one can connect to the patient database DP to acquire, in addition to the patient's personal data, also the anamnesis data necessary for the next processing steps.

In this menu, a list of signs and symptoms observed and/or reported during the medical examination is associated with the selected patient.

Signs and symptoms can be selected in many ways. The simplest way is to use a search engine within the symptom **DB**: the user writes the reported symptom or sign in a dialog box and, based on this, the search engine will provide a list of signs and symptoms similar to the one just entered, from which the user will be allowed to select the one that he/she considers as most appropriate to describe the patient's situation.

At the end of the selection, the user will have a list of signs and symptoms that, together with the patient's anamnesis information (already in the database), will allow returning to the Home menu and processing the list of the most likely pathologies.

### *Patient data* menu (procedure).

available options:

> **Patient selection**
> displays the patient's personal information
> **Sign and symptom selection**
> signs and symptoms are entered by selecting them in the **DB**
> **Back**
> returns to the *Home* menu

the output displays:

> the patient's personal information (once selected)
> the list of selected signs and symptoms observed in the patient

## *SYMPTOM LIST* MENU

This menu displays all signs and symptoms associated with a predefined pathology.

The number of signs and symptoms displayed can be at most equal to the NuMaxSint variable (20 by default); among these, those already observed by the physician (i.e. already selected) are highlighted in bold. It is thus possible to verify that all relevant symptoms have been considered for the pathology of interest, and verify the presence of any further typical symptoms not yet detected/reported.

If there are any, the physician can then enter those new symptoms that were not entered during the first investigation step. The list of the most likely pathologies will be updated accordingly upon returning to the Home menu.

### *Symptom list* menu (procedure).

available options:

> **Configuration**
> *opens the **CPDA** menu*
> **Back**

returns to the _Home_ menu

the output displays the list of 20 (default value of the NuMaxSint variable) signs and symptoms for that pathology

The selected signs and symptoms are displayed in bold.
Others can be selected (or unselected) by clicking on them.

## SELECTION OF RELEVANT SYMPTOMS MENU

This option allows detecting important signs and symptoms that were previously ignored, in order to refine the differential diagnosis among the most likely hypotheses.
From the first 5 (default value of the NuSePa variable) diagnostic hypotheses in the list, all symptoms not reported during the first investigation step are extracted. Only the first 5 symptoms are displayed (default value of SiPr) with the highest absolute LR (likelihood ratio) (maximum LR of a symptom among the most likely pathologies).
If there are any, the physician can then enter those new symptoms that were not entered during the first investigation step. The list of the most likely pathologies will be updated accordingly upon returning to the Home menu.

### _Selection of relevant symptoms_ menu (procedure).

available options:

_**Configuration**_
_opens the_ **CPDA** _menu_
**Back**
returns to the _Home_ menu

the output displays a list that may contain at most NuSePa * SiPr signs and symptoms.

The selected signs and symptoms are displayed in bold.
Others can be selected (or unselected) by clicking on them.

## ADD RARE DISEASE MENU

This option allows verifying that a pathology that is not very likely (rare disease) has not been neglected, although a highly relevant symptom was detected for that disease. Among all symptoms reported during the first investigation step, the system will display the first 10 symptoms (default value of SiPiuP) having the highest LR and the pathology for which such value is highest.
If necessary, the physician can add low-probability pathologies to the list of rare diseases already considered, and then display them in the next menu.

### _Add rare disease_ menu (procedure).

available options:

**Configuration**
_opens the_ **CPDA** _menu_
**Back**
returns to the _Home_ menu

the output displays a list of SiPiuP (10 by default) signs and symptoms with which the pathology having the highest LR is associated (via interrogation of the **DB**); the already selected signs and symptoms and pathology are displayed in bold. Others can be selected (or unselected) by clicking on them.

**SIT MENU**

Menu for semi-automatic interactive selection of tests with high diagnostic effect. To each pathology in the selected list (LPS: list of selected pathologies), a specific set of diagnostic tests corresponds.

Of all these tests, only those having a pre-test probability lower than or equal to 70% (default value of the SoSePa_sup variable) are taken into account. The tests thus selected make up the so-called TAMS set (tests associated with selected diseases).

From the TAMS set containing all relevant tests, the system first selects those having, in absolute terms (i.e. for all selected pathology) an LR above an *effectiveness* threshold SoEf (2 by default), and then selects from the latter and displays those which, for at least one of the selected pathologies, exceed the post-test probability value of 70% (default value of the SoPPT variable, which is in any case smaller than or equal to SoSePa_sup).

The physician can then select/unselect the TAMS tests as considered appropriate.

*SIT* **menu (procedure).**

available options:

**Back**
returns to the previous menu
**Configure appropriate diagnostic paths**
opens the *CPDA* menu

the output displays the list of the diagnostic tests belonging to the TAMS set and having LR > SoEf and post-test probability > SoPPT.

The tests can be selected/unselected by clicking on them.
The selected tests are displayed in bold.

**SMT MENU**

The menu for manually selecting the tests for the pathology selected in the previous menu displays all relevant tests sorted by relative likelihood ratio, so that the physician can select one or more diagnostic tests at will. In some cases, even a test not exceeding the effectiveness threshold may be chosen and entered into the TAMS set.

*SMT* **menu (procedure).**

available options:

**Back**
includes the selected tests into the TAMS set (if not already present) returns to the previous menu
**Configure appropriate diagnostic paths**
opens the *CPDA* menu

the output displays the list of diagnostic tests.

The tests can be selected/unselected by clicking on them.
The selected tests are displayed in bold.

**CPDA MENU**

In the Appropriate diagnostic path configuration it is possible to display, and possibly modify, the values of the system variables.

*CPDA* menu (procedure).

available options:

**NuMaxSint**

20 by default;
maximum number of signs and symptoms that can be selected per pathology
it is used in the *Symptom list* menu

**NuPaLis**

10 by default; number of pathologies in the list.
maximum number of pathologies listed in the menu
*home* from **LP** procedure

**NuSePa**

5 by default; maximum number of selected pathologies.
Number of most likely pathologies considered in the
*Selection of relevant symptoms* menu

**SiPr**

10 by default; probable symptoms.
maximum number of most likely signs and symptoms ignored per pathology;
it is used in the
*Selection of relevant symptoms* menu

**SiPiuP**

10 by default; most likely symptoms.
Maximum number of pathologies for each one of which the selected signs and symptoms have the highest
value of LR.
It is used in the *Add rare disease* menu

**SoSePa_inf**
40% by default; lower pathology selection threshold; threshold of the probability of a pathology, above which
that pathology will be included in the LPP list of the pathologies automatically selected in the *Pathology selection
for differential diagnosis* menu. This is the lower limit of the diagnostic uncertainty interval, given by the difference
SoSePa_sup - SoSePa_inf.
**SoSePa_sup**

70% by default; upper pathology selection threshold. Upper limit of the diagnostic uncertainty interval, given
by the difference SoSePa_sup - SoSePa_inf.
Only diagnostic tests associated with pathologies having pre-test probabilities within this interval will be
taken into account in the *Diagnostic test selection* menu.

**SoEf**

2 by default; effectiveness threshold
value of the LR of a diagnostic test above which a diagnostic test can be selected by the physician in order
to process a diagnostic path. It is used in the *SIT* menu

**SoPPT**

70% by default; post-test probability threshold.
Threshold beyond which a test can be included by the physician into the list of diagnostic paths. SoPPt must be <= SoSePa_sup.
It is used in the *SIT* menu

**Te: test access wait time**
the use of this variable can be selected/unselected for computing the appropriateness
**C : test cost index**
the use of this variable can be selected/unselected for computing the appropriateness
**R: test risk index**
the use of this variable can be selected/unselected for computing the appropriateness
**To: test tolerability index**
the use of this variable can be selected/unselected for computing the appropriateness
**Show appropriateness values**
for selecting/unselecting the possibility of explicitly displaying the appropriateness values in the output of the *Diagnostic test selection* menu
**Back**

returns to the previous menu
the output will display the updated value of each variable.

**LP procedure - description**

The LP procedure builds the list of most likely pathologies (LPP) by using a NAIVE BAYES model (e.g. as described in Wagholikar et al., op. cit.).

The procedure starts from the prevalence, expressed in terms of odds, of the pathology, i.e. from the epidemiological datum.

In statistics, the term "odds" refers to the ratio between the probability "p" of an event and the probability that such event will not occur (i.e. the probability (1-p) of the complementary event). Odds_prevalenza indicates the ratio between the probability that the patient is suffering from a pathology, based on demographic and age-related considerations (prevalence), and the probability that the patient is not suffering from that pathology (1-prevalence). The prevalence of the pathology M being defined as P(M), it follows that odds_prevalenza = P(M) / (1-P(M)).

A likelihood ratio LR is associated with each symptom, sign, executed test and anamnesis datum, which is given by:

$$LR = P(S|M) / P(S|{\sim}M)$$

i.e. by the conditional probability of finding the symptom (S) in a diseased patient (M) divided by the conditional probability of finding the same symptom in a patient not suffering from that specific disease ($\sim$M). The global LRg of a set of signs, symptoms, etc. is given by the product of the individual LR's.

It is now possible to estimate the probability that a patient with those symptoms, signs, etc. has the disease M prior to executing any further diagnostic tests (pre-tests); in terms of odds, this is written as:

$$odds\_pre\_test = LRg * odds\_prevalenza$$

and this can, in general, be expressed again in terms of probability P, knowing that P = odds / (1+ odds).

The above-described formula for the calculation of odds_pre_test can be easily and quickly implemented and, as aforesaid, from the latter one can calculate the probability P_pre_test. As a slightly more complex alternative, one may directly compute the pre-test probability for a succession of n symptoms in an iterative or recursive manner. In this case, the following relation is used:

$$P_i = (LR * P_{i-1}) / (LR * P_{i-1} + K_{i-1})$$

where $K_{i-1}=(1 - P_{i-1})$
$P_i$ is the pre-test probability of the first i symptoms; where i ranges from 1 to all n symptoms, and $P_0$ is the prevalence

of the pathology. Of course, P_pre_test = $P_n$ Although this implementation appears to be more complex, it allows the use of simple variants of the strictly Bayesian model. For example, different formulations of $K_{i-1}$ can be adopted, such as the following two:

$$K_{i-1} = (1 - P_0) \text{ or } K_{i-1} = (1 - P_{i-1})^{LR}.$$

The basic idea of these latter two formulations is to reduce the pre-test probabilities obtained with the Bayes formula, which considers all system variables as independent of one another.

**LP procedure**

for (each pathology of the "Diseases" list from **BDC**)

P0 =  prevalence of the pathology

ODDS = P0 /(1- P0)   /* prevalence expressed in terms of odds  */

LRg = 1    /*global likelihood ratio for all data associated with the patient*/

for (each symptom, sign, anamnesis datum, executed test)

read the corresponding LR (likelihood ratio)

LRg = LRg * LR

ODDSpre = LRg * ODDS

PpreTest=ODDSpre/(ODDSpre+1)  /* PpreTest is the pre-test probability */

stores the LPP list of each pathology with the respective PpreTest

end

**OPDA procedure** - **description**

The procedure processes the output of the appropriate diagnostic path by using the same NAIVE BAYES model and the same formulae as shown in the LP procedure.

Let LR be the likelihood ratio associated with a generic diagnostic test; the estimated probability (expressed in terms of odds) of the pathology M, if the test (post-test) has a positive outcome, will be given by:

$$odds\_post\_test = LR * odds\_pre\_test$$

and this can then be expressed again in terms of probability P, knowing that P = odds / (1+ odds).

This probability can be evaluated for every single test and for the full test set.

The procedure also associates with each test its respective appropriateness index IA, defined as:

$$IA = To / (C * Te * R)$$

where To is test tolerability index; C is the test cost index; Te is the test access wait time index; R is the index of the maximum between the intrinsic risk and the relative risk of the test.

The higher the appropriateness, the greater the value of the IA index.

The global appropriateness index ($IA_G$) of all the selected diagnostic tests is computed as follows:

$$IA_G = To_G / (C_G * Te_G * R_G)$$

where $To_G$ is the minimum among all tolerability indexes; $C_G$ is the sum of the individual costs for each test; $Te_G$ is the maximum among all wait times; $R_G$ is the maximum among all risk indexes.

The physician may decide to define an alternative path (by selecting a different set of tests); the system will keep in memory the previous path with the respective appropriateness indexes, so that the physician can compare different paths.

For the computation of odds_post_test, the same considerations apply as those made while commenting on the LP procedure for the computation of the probability P_pre_test in regard to the statistical independence of the variables associated with signs, symptoms and tests. The above-described variants of the strictly Bayesian computation apply to this case as well.

**OPDA procedure**

```
for (each pathology of the LPS list from BDC)

    P0 = PpreTest

    /* previously computed pre-test probability of the pathology */

    ODDS = P0 /(1- P0)

    LRg = 1   /*global likelihood ratio for all tests*/

    for (each test belonging to the TAMS set )

        read the corresponding LR (likelihood ratio)

        LRg = LRg * LR

        read Te /*  access wait time index of the test */

        read C /*  cost index of the test */

        read Ri /*  intrinsic risk index of the test */

        read Rr /*  relative risk index of the test */

        determine R /* as the maximum between Ri and Rr of the test */

        read To /*  tolerability index of the test */

        compute CG as the sum of all C indexes

        determine TeG as the maximum of the individual Te indexes

        determine RG as the maximum of the individual R indexes

        determine ToG as the minimum of the individual To indexes

        Appropriateness = To / (C*Te*R) /* appropriateness index of the

        test */

        IAG= ToG / (CG*TeG* RG)  /* global appropriateness of all tests */
```

$$K = LR * ODDS$$

$$PpostTest = K/(1 + K) \text{ /* post-test probability for each test */}$$

$$ODDSpostG = LRg * ODDS$$

/* compute the global post-test probability */

$$PpostTestG = ODDSpostG / (1 + ODDSpostG)$$

for (each pathology in the LPS list from BDC)

saves and displays:

the name of the pathology and its respective PpreTest

saves and displays, in decreasing appropriateness order,

each symptom with its respective PpostTest

and (optionally) the appropriateness index

the global post-test probability: PpostTestG

the global appropriateness of all tests (if the option is on)

end

**Glossary**

BDC: Base Di Conoscenza (Knowledge base)
C : cost of the diagnostic test (variable)
CPDA: Configurazione Percorso Diagnostico Appropriato (Appropriate diagnostic path configuration) (menu)
DB: database
DP: dati paziente (patient data)
LMR: lista delle Malattie Rare (List of rare diseases)
LP: procedure for the computation of the probabilities of the diagnostic hypotheses (procedure)
LPA: Lista Patologie Aggiunte (List of manually added pathologies)
LPP: Lista di Patologie più Probabili (List of most likely pathologies)
LPS: Lista delle Patologie Selezionate (List of selected pathologies)
LR: Likelihood ratio
NuMaxSint Maximum number of signs and symptoms that can be selected per pathology (variable)
NuPaLis Number of pathologies in the list (variable)
NuSePa: Maximum number of selected pathologies (variable)
OPDA: Output Percorso Diagnostico Appropriato (Appropriate diagnostic path output)
OPDA: procedure for processing the PDA output (procedure)
R: Risk associated with the test (variable)
Ri: Intrinsic risk of the test (variable)
Rr: Relative risk of the test (variable)
SiPiuP: Most likely symptoms (variable)
SiPr: Probable symptoms (variable)
SIT: Interactive diagnostic test selection (menu)
SMT: Manual diagnostic test selection (menu)
SoEf: Effectiveness threshold (variable)
SoPPT: Post-test probability threshold (variable)
SoSePa_inf: Lower pathology selection threshold (variable)
SoSePa _sup: Upper pathology selection threshold (variable)
TAMS: set of tests associated with the selected diseases

Te: diagnostic test access time (variable)
To: test tolerability index (variable)

The present invention can advantageously be implemented through a computer program, which comprises coding means for implementing one or more steps of the method when said program is executed by a computer. It is therefore understood that the protection scope extends to said computer program as well as to computer-readable means that comprise a recorded message, said computer-readable means comprising program coding means for implementing one or more steps of the method when said program is executed by a computer.

The above-described example of embodiment may be subject to variations without departing from the protection scope of the present invention, including all equivalent designs known to a man skilled in the art.

The elements and features shown in the various preferred embodiments may be combined together without however departing from the protection scope of the present invention. From the above description, those skilled in the art will be able to produce the object of the invention without introducing any further construction details.

**Claims**

1. Differential diagnosis apparatus adapted for medical applications in order to determine an optimal sequence of diagnostic tests for identifying a pathology by adopting diagnostic appropriateness criteria, comprising a system of processors, in turn comprising:

   - a first updatable database containing patients' data;
   - a second updatable relational database containing identification data of pathologies, symptoms, clinical signs, identification data of diagnostic tests, and data relating to the appropriateness parameters of said diagnostic tests for defining a list of diagnostic hypotheses (pathologies);
   - means adapted to determine said optimal sequence of diagnostic tests for identifying a pathology, said means comprising an inferential computation engine, which determine for each diagnostic hypothesis (pathology), based on data contained in said first and second databases, said optimal sequence of diagnostic tests with associated global and single indexes of appropriateness and probability that a patient is suffering from that pathology, said differential diagnosis apparatus being **characterized in that** said means are adapted to:

   - determine said optimal sequence of diagnostic tests;
   - determine said appropriateness index IA of a single diagnostic test defined as: $IA = To / (C * Te * R)$, where Te is the test access wait time index; C is the test cost index; R is the index of the maximum between the intrinsic risk and the relative risk of the test; To is the test tolerability index;
   - determine said global appropriateness index of a sequence of diagnostic tests $IA_G=To_G/(C_G*Te_G*R_G)$, where $To_G$ is the minimum among all tolerability indexes of all tests in the sequence, $C_G$ is the sum of the single costs of each test in the sequence, $Te_G$ is the maximum among all wait times, $R_G$ is the maximum among all risk indexes of the sequence,

   wherein tolerability indexes reflect the tolerability of each test for the patient.

2. Diagnosis apparatus according to claim 1, wherein said means adapted to determine said optimal sequence of diagnostic tests are adapted to, through said inferential computation engine:

   - determine, starting from said data contained in said first and second databases, a pre-test probability index of the possible diagnostic hypotheses (pathologies);
   - determine, starting from said data contained in said first and second databases and from said pre-test probability index, a post-test probability index of the possible diagnostic hypotheses (pathologies) conditional upon the execution of said optimal sequence of diagnostic tests;
   - associate an appropriateness index with each test of said optimal sequence;
   - associate an appropriateness index with said optimal sequence of diagnostic tests.

3. Diagnosis apparatus according to claim 2, wherein said means adapted to determine said optimal sequence of diagnostic tests are adapted to determine said pre-test probability Pre(M | {**DP**}) and said post-test probability Post(M | {**DP**} U {T} ) on the basis of said inferential computation of respective pre-test and post-test probabilities of finding said pathology M conditional upon, respectively, the set of the patient's identification data contained in said first database {**DP**} and the association of said patient's identification data {**DP**} with the set of said tests {T} of said optimal sequence of diagnostic tests.

4.  Diagnosis apparatus according to claim 3, wherein said means adapted to determine said optimal sequence of diagnostic tests comprise means adapted to determine said pre-test probability Pre(M | {**DP**}) in a manner such that:

> - with each symptom a likelihood ratio LR is associated, which is given by:

$$LR = P(S|M) / P(S| \sim M)$$

> i.e. by the conditional probability P(S|M) of finding the symptom (S) in a patient suffering from a pathology (M) divided by the conditional probability P(S|~M) of finding the same symptom in a patient not suffering from said pathology (~M);

> - computing a global likelihood ratio LRg given by the product of the likelihood ratios LR of each pathology;
> - computing said relative pre-test probability Pre(M | {**DP**}) in terms of odds first:

$$odds\_pre\_test = LRg * odds\_prevalenza$$

> where odds_prevalenza = P(M) / (1 - P(M)), where P(M) is the prevalence of the pathology M.
> from which said pre-test probability Pre(M | {**DP**}) is given by:

$$Pre(M | \{\mathbf{DP}\}) = odds / (1 + odds).$$

5.  Diagnosis apparatus according to claim 4, wherein said means adapted to determine said optimal sequence of diagnostic tests comprise means adapted to determine said post-test probability Post(M | {**DP**} U {T} ) in a manner such that:

$$odds\_post\_test = LR * odds\_pre\_test$$

from which said post-test probability Post(M | {**DP**} U {T} ) is given by:

$$Post(M | \{\mathbf{DP}\} \ U \ \{T\} ) = odds\_post\_test / (1 + odds\_post\_test).$$

6.  Diagnosis apparatus according to claim 3, wherein said means adapted to determine said optimal sequence of diagnostic tests comprise means adapted to determine said pre-test probability Pre(M | {**DP**}) for a succession of symptoms in an iterative or recursive manner, by using the following relation:

$$P_i = (LR * P_{i-1}) / ( LR * P_{i-1} + K_{i-1} ) \quad where \ K_{i-1} = (1 - P_{i-1})$$

$P_i$ is the pre-test probability of the first i symptoms; where i ranges from 1 to all n symptoms, and $P_0$ is the prevalence of the pathology;

> - then determine said pre-test probability Pre(M | {**DP**}) = $P_n$.

7.  Diagnosis apparatus according to claim 1, comprising software modules implemented in storage devices readable and executable by a computer equipped with input/output devices, implemented in (physical or virtual) computation clusters, and remotely executable and implemented as Apps for mobile devices (tablets, smartphones, notebook PCs).

**Patentansprüche**

1. Differenzialdiagnostische Einrichtung, die für medizinische Anwendungen angepasst ist, um eine optimale Abfolge von Diagnosetests zum Identifizieren einer Pathologie zu ermitteln, durch Übernehmen von diagnostischen Relevanzkriterien, umfassend ein System von Prozessoren, das wiederum umfasst:

   - eine erste aktualisierbare Datenbank, die Patientendaten enthält;
   - eine zweite aktualisierbare Beziehungsdatenbank, die Identifikationsdaten von Pathologien, Symptomen, klinischen Zeichen, Identifikationsdaten von Diagnosetests und Daten bezüglich der Relevanzparameter der Diagnosetests zum Definieren einer Liste von diagnostischen Hypothesen (Pathologien) zu definieren;
   - Mittel, die angepasst sind, die optimale Abfolge von Diagnosetests zum Identifizieren einer Pathologie zu ermitteln, wobei die Mittel eine Rückschlussberechnungsengine umfassen, die für jede diagnostische Hypothese (Pathologie) basierend auf Daten, die in der ersten und zweiten Datenbank enthalten sind, die optimale Abfolge von Diagnosetests mit zugehörigen globalen und einzelnen Indizes von Relevanz und Wahrscheinlichkeit ermitteln, dass ein Patient an dieser Pathologie leidet, wobei die Differenzialdiagnostische Einrichtung **dadurch gekennzeichnet ist, dass** die Mittel angepasst sind zum:

      - Ermitteln der optimalen Abfolge von Diagnosetests;
      - Ermitteln des Relevanzindexes IA eines einzelnen Diagnosetests, der definiert ist als: $IA = To/(C \cdot Te \cdot R)$, wo Te der Testzugriffswartezeitindex ist; C der Testkostenindex ist; R der Index des Maximums zwischen dem intrinsischen Risiko und dem relativen Risiko des Tests ist; To der Testverträglichkeitsindex ist;
      - Ermitteln des globalen Relevanzindexes einer Abfolge von Diagnosetests $IA_G = To_G/(C_G \cdot Te_G \cdot R_G)$, wo $To_G$ das Minimum unter allen Verträglichkeitsindizes aller Tests in der Abfolge ist, $C_G$ die Summe der einzelnen Kosten von jedem Test in der Abfolge ist, $Te_G$ das Maximum unter allen Wartezeiten ist, $R_G$ das Maximum unter allen Risikoindizes der Abfolge ist,

   wobei Verträglichkeitsindizes die Verträglichkeit jedes Tests für den Patienten widerspiegeln.

2. Diagnostische Einrichtung nach Anspruch 1, wobei die Mittel, die angepasst sind, die optimale Abfolge von Diagnosetests zu ermitteln, durch die Rückschlussrechenengine angepasst sind zum:

   - Ermitteln, beginnend bei den Daten, die in der ersten und zweiten Datenbank enthalten sind, eines Vortestwahrscheinlichkeitsindexes der möglichen diagnostischen Hypothesen (Pathologien);
   - Ermitteln, beginnend bei den Daten, die in der ersten und zweiten Datenbank enthalten sind, und aus dem Vortestwahrscheinlichkeitsindex eines Nachtestwahrscheinlichkeitsindexes der wahrscheinlichen diagnostischen Hypothesen (Pathologien) abhängig von der Ausführung der optimalen Abfolge der Diagnosetests;
   - Verknüpfen eines Relevanzindexes mit jedem Test der optimalen Abfolge;
   - Verknüpfen eines Relevanzindexes mit der optimalen Abfolge von Diagnosetests.

3. Diagnostische Einrichtung nach Anspruch 2, wobei die Mittel, die zum Ermitteln der optimalen Abfolge von Diagnosetests angepasst sind, angepasst sind, die Vortestwahrscheinlichkeit Pre((M|{**DP**}) und die Vortestwahrscheinlichkeit Post(M|{**DP**}U{T}) auf der Basis der Rückschlussberechnung jeweiliger Vortest- und Nachtestwahrscheinlichkeiten, die Pathologie M zu finden, abhängig von dem Satz von Patientenidentifizierungsdaten, die in der ersten Datenbank {**DP**} enthalten sind, beziehungsweise der Verknüpfung der Patientenidentifizierungsdaten {**DP**} mit dem Satz von Tests {T} der optimalen Abfolge von Diagnosetests zu ermitteln.

4. Diagnostische Einrichtung nach Anspruch 3, wobei die Mittel, die angepasst sind, die optimale Abfolge von Diagnosetests zu ermitteln, Mittel umfassen, die angepasst sind, die Vortestwahrscheinlichkeit Pre((M|{**DP**}) auf so eine Weise zu ermitteln, dass:

   - mit jedem Symptom ein Likelihood-Verhältnis LR verknüpft ist, das vorgegeben ist durch:

$$LR = P(S|M)/P(S|{\sim}M)$$

   d.h. durch die abhängige Wahrscheinlichkeit P(S|M), das Symptom (S) in einem Patienten zu finden, der an einer Pathologie (M) leidet, geteilt durch die abhängige Wahrscheinlichkeit P(S|~M), dasselbe Symptom in einem Patienten zu finden, der nicht an der Pathologie (~M) leidet;

- Berechnen eines globalen Likelihood-Verhältnisses LRg, das durch die Produkt des Likelihood-Verhältnisse LR jeder Pathologie erhalten ist;
- Berechnen der relativen Vortestwahrscheinlichkeit Pre((M|{**DP**}) im Sinne von Quoten zuerst:

$$odds\_pre\_test = LRg*odds\_prevalenza$$

wo odds_prevalenza = P(M)/(1-P(M)), wo P(M) die Prävalenz der Pathologie M ist, aus der die Vortestwahrscheinlichkeit Pre((M|{**DP**}) erhalten ist durch:

$$Pre((M|\{\textbf{DP}\}) = odds/(1+odds).$$

**5.** Diagnostische Einrichtung nach Anspruch 4, wobei die Mittel, die angepasst sind, die optimale Abfolge von Diagnosetests zu ermitteln, Mittel umfassen, die angepasst sind, die Nachtestwahrscheinlichkeit Post((M|{**DP**}U{T}) auf so eine Weise zu ermitteln, dass:

$$odds\_post\_test=LR*odds\_pre\_test$$

aus dem Nachtestwahrscheinlichkeit Post((M|{**DP**}U{T}) erhalten ist durch:

$$Post((M|\{\textbf{DP}\}U\{T\})=odds\_post\_test/1+odds\_post\_test).$$

**6.** Diagnostische Einrichtung nach Anspruch 3, wobei die Mittel, die angepasst sind, die optimale Abfolge von diagnostischen Tests zu ermitteln, Mittel umfassen, die angepasst sind, die Vortestwahrscheinlichkeit Pre((M|{**DP**}) für eine Aufeinanderfolge von Symptomen auf eine wiederholende oder wiederkehrende Weise zu ermitteln, unter Verwendung der folgenden Beziehung:

$$P_i=(LR*P_{i-1})/(LR*P_{i-1}+K_{i-1}), \text{ wo } K_{i-1}=(1-P_{i-1})$$

$P_i$ ist die Vortestwahrscheinlichkeit der ersten i Symptome; wo i von 1 bis alle n Symptome reicht und $P_0$ ist die Prävalenz der Pathologie;

- dann Ermitteln der Vortestwahrscheinlichkeit Pre((M|{**DP**}) = $P_n$.

**7.** Diagnostische Einrichtung nach Anspruch 1, umfassend Softwaremodule, die in Speichervorrichtungen implementiert sind, die von einem Computer lesbar und ausführbar sind, der mit Eingangs-/Ausgangsvorrichtungen ausgestattet ist, die in (physischen oder virtuellen) Rechenclustern implementiert sind und aus der Ferne ausführbar und als Apps für Mobilvorrichtungen (Tablets, Smartphones, Notebook-PCs) implementiert sind.

## Revendications

**1.** Appareil de diagnostic différentiel adapté à des applications médicales afin de déterminer une séquence optimale de tests de diagnostic afin d'identifier une pathologie en adoptant des critères de pertinence de diagnostic, comprenant un système de processeurs, comprenant à leur tour :

- une première base de données actualisable contenant des données de patients ;
- une seconde base de données relationnelles actualisable contenant des données d'identification de pathologies, de symptômes, de signes cliniques, des données d'identification de tests de diagnostic et des données en rapport avec les paramètres de pertinence desdits tests de diagnostic pour définir une liste d'hypothèses de diagnostic (pathologies) ;
- des moyens qui sont à même de déterminer ladite séquence optimale de tests de diagnostic afin d'identifier une pathologie, lesdits moyens comprenant un moteur de calcul inférentiel, qui détermine pour chaque hypothèse de diagnostic (pathologie), sur la base de données contenues dans lesdites première et seconde bases de données, ladite séquence optimale de tests de diagnostic avec des index globaux et individuels associés de pertinence et de probabilité qu'un patient souffre de cette pathologie, ledit appareil de diagnostic différentiel étant **caractérisé en ce que** lesdits moyens sont à même de :

- déterminer ladite séquence optimale de tests de diagnostic ;
- déterminer ledit index de pertinence IA d'un test de diagnostic individuel défini par IA = To / (C * Te * R), où Te est l'index de temps d'attente pour l'accès aux tests ; C est l'index de coût de tests ; R est l'index du maximum entre le risque intrinsèque et le risque relatif du test ; To est l'index de capacité de tolérance des tests ;
- déterminer ledit index de pertinence globale d'une séquence de tests de diagnostic $IA_G = To_G/(C_G * Te_G * R_G)$, où $To_g$ est le minimum parmi tous les index de capacité de tolérance de tous les tests de la séquence, $C_G$ est la somme des coûts individuels de chaque test de la séquence, $Te_G$ est le maximum parmi tous les temps d'attente, $R_G$ est le maximum parmi tous les index de risque de la séquence,

dans lequel les index de capacité de tolérance reflètent la capacité de tolérance de chaque test pour le patient.

2. Appareil de diagnostic selon la revendication 1, dans lequel lesdits moyens qui sont à même de déterminer ladite séquence optimale de tests de diagnostic sont à même, via ledit moteur de calcul inférentiel, de :

- déterminer, en partant desdites données contenues dans lesdites première et seconde bases de données, un index de probabilité de pré-test des hypothèses de diagnostic possibles (pathologies) ;
- déterminer, en partant desdites données contenues dans lesdites première et seconde bases de données et à partir dudit index de probabilité de pré-test, un index de de probabilité de post-test des hypothèses de diagnostic possibles (pathologies) conditionnelles lors de l'exécution de ladite séquence optimale de tests de diagnostic ;
- associer un index de pertinence à chaque test de ladite séquence optimale ;
- associer un index de pertinence à ladite séquence optimale de tests de diagnostic.

3. Appareil de diagnostic selon la revendication 2, dans lequel lesdits moyens qui sont à même de déterminer ladite séquence optimale de tests de diagnostic sont à même de déterminer la probabilité de pré-test Pré(M | {**DP**}) et ladite probabilité de post-test Post(M | {**DP**} U {T} ) sur la base dudit calcul inférentiel de probabilités de pré-test et de post-test respectives de trouver ladite pathologie M conditionnelle lors, respectivement, de l'ensemble des données d'identification du patient contenues dans ladite première base de données {**DP**} et de l'association desdites données d'identification du patient {**DP**} avec l'ensemble desdits tests {T} de ladite séquence optimale de tests de diagnostic.

4. Appareil de diagnostic selon la revendication 3, dans lequel les moyens qui sont à même de déterminer ladite séquence optimale de tests de diagnostic comprennent des moyennes qui sont à même de déterminer ladite probabilité de pré-test Pré(M | {**DP**}) d'une manière telle que :

- avec chaque symptôme, un rapport de vraisemblance LR soit associé, qui est donné par :

$$LR = P(S|M) / P(S| \sim M)$$

c'est-à-dire par la probabilité conditionnelle P(S|M) de trouver le symptôme (S) chez un patient souffrant d'une pathologie (M) divisée par la probabilité conditionnelle P(S|~M) de trouver le même symptôme chez un patient ne souffrant pas de ladite pathologie (~M) ;
- le calcul d'un rapport de vraisemblance global LRg donné par le produit des rapports de vraisemblance LR de chaque pathologie ;
- le calcul de ladite probabilité de pré-test relative Pré(M | {**DP**}) en termes de nombres impairs d'abord :

$$odds\_pré\_test = LRg * odds\_prevalenza$$

où odds_prevalenza = P(M) / (1 - P(M)), où P(M) est la prévalence de la pathologie M, à partir duquel ladite probabilité de pré-test Pré(M | {**DP**}) est donnée par :

$$Pré(M) \mid \{DP\}) = odds \ / \ (1+odds).$$

5. Appareil de diagnostic selon la revendication 4, dans lequel lesdits moyens qui sont à même de déterminer ladite séquence optimale de tests de diagnostic comprennent des moyens qui sont à même de déterminer ladite probabilité de post-test $Post(M \mid \{DP\} \cup \{T\})$ d'une manière telle que :

$$odds\_post\_test = LR * odds\_pré\_test,$$

à partir duquel ladite probabilité de post-test $Post(M \mid \{DP\} \cup \{T\})$ est donnée par

$$Post(M) \mid \{DP\} \cup \{T\}) = odds\_post\_test \ / \ (1+odds\_post\_test).$$

6. Appareil de diagnostic selon la revendication 3, dans lequel lesdits moyens qui sont à même de déterminer ladite séquence optimale de tests de diagnostic comprennent des moyens qui sont à même de déterminer ladite probabilité de pré-test $Pré(M \mid \{DP\})$ pour une succession de symptômes de manière itérative ou récursive en utilisant la relation suivante :

$$P_i = (LR * P_{i-1}) \ / \ (LR * P_{i-1} + K_{i-1})$$

où $K_{i-1} = (1 - P_{i-1})$, $P_i$ est la probabilité de pré-test des $i$ premiers symptômes ; où $i$ se situe dans la plage de 1 à la totalité des $n$ symptômes et $P_0$ est la prévalence de la pathologie ;

- puis de déterminer ladite probabilité de pré-test $Pré(M) \mid \{DP\}) = P_n$.

7. Appareil de diagnostic selon la revendication 1, comprenant des modules de logiciels mis en œuvre dans des dispositifs de stockage lisibles et exécutables par un ordinateur équipé de dispositifs d'entrée/sortie, mis en œuvre dans des groupes de calcul (physiques ou virtuels) et exécutables et mis en œuvre à distance sous forme d'appareils pour dispositifs mobiles (tablettes, smartphones, ordinateurs portables).

**FIG. 1**

**FIG. 2**

```
┌──────────────────────────────────┐
│          HOME menu               │
│                                  │
│     Options:  ┆ ─────────────────┼──────▶  • Patient data menu
│                                  │         • Symptom list menu (in turn calling the
│     Output:                      │           Sign and symptom selection menu)
│  Calls the LP routine and        │
│  displays the LPP+LMR list       │         • Relevant symptom selection menu
│                                  │
│                                  │         • Add rare disease menu
└──────────────────────────────────┘         • CPDA menu
                 │
                 ▼
┌──────────────────────────────────┐
│  Pathology selection for         │
│  differential diagnosis menu     │
│                                  │
│                                  │
│    ⎸Options:  ───────────────────┼──────▶  • Automatic selection
│                                  │         • Manual selection
│     Output:                      │         • Modify list
│  displays the LPS=LPP+LMR+LPA    │         • Back to Home menu
│  list                            │         • CPDA menu
│                                  │
└──────────────────────────────────┘
                 │
                 ▼
┌──────────────────────────────────┐
│  Diagnostis test selection menu  │
│                                  │
│                                  │
│    Options:  ┆ ──────────────────┼──────▶  • SIT menu
│                                  │         • SMT menu
│     Output:                      │         • Back to previous menu
│  Calls the OPDA procedure        │         • Save
│                                  │         • Load
│                                  │         • Print
│                                  │         • CPDA menu
└──────────────────────────────────┘
```

# FIG. 3

32

## REFERENCES CITED IN THE DESCRIPTION

### Patent documents cited in the description

- WO 2012122198 A1
- US 2013185093 A1

- US 7076437 B1

### Non-patent literature cited in the description

- Crossing the quality chasm: a new health system for the 21st century. National Academy Press, 2001
- FERRANTE DI RUFFANO L. ; HYDE C.J. ; MCCAFFERY K.J. ; BOSSUYT P.M.M. ; DEEKS J.J. Assessing the value of diagnostic tests: a framework for designing and evaluating trials. *British Medical Journal,* 2012, 344-352
- WAGHOLIKAR, K. B. ; SUNDARARAJAN, V. ; DESHPANDE, A. W. Modeling Paradigms for Medical Diagnostic Decision Support: A Survey and Future Directions. *J. Medical Systems,* 2012, vol. 36 (5), 3029-49
- EL-KAREH R. ; HASAN O. ; SCHIFF G.D. Use of health information technology to reduce diagnostic errors. *BMJ Quality & Safety,* 2013, vol. 22 (2), ii40-ii51

- BARNETT G.O. ; CIMINO J.J. ; HUPP J.A. ; HOFFER E.P. DXPlain - An evolving diagnostic decision-support system. *JAMA,* 1987, vol. 258 (1), 67-74
- RAMNARAYAN P ; TOMLINSON A ; RAO A. ; COREN M. ; WINROW A. ; BRITTO J. ISABEL. A web-based differential diagnostic aid for paediatrics: results from an initial performance evaluation. *Archives of Disease in Childhood,* 2003, vol. 88, 408-413
- KAWAMOTO K. ; HOULIHAN C.A. ; BALAS E.A. ; LOBACH D.F. Improving clinical practice using clinical decision support systems: a systematic review of trials to identify features critical to success. *BMJ,* 2005, 330-337
- NADKARNI P.M. ; OHNO-MACHADO L. ; CHAPMAN W.W. Natural language processing: an introduction. *Journal of the American Medical Informatics Association,* 2011, vol. 18, 544-551